# EUROPEAN PATENT APPLICATION

(11) **EP 4 848 310 A1**
(43) Date of publication of application: **02.12.2026**
(21) Application number: 25204476.3
(22) Date of filing: 24.09.2025
(51) Int. Cl.: G16H 70/20, G16H 80/00

(54) **COMPUTER-IMPLEMENTED METHOD AND SYSTEM FOR MANAGING A DIGITAL ACADEMIC PLATFORM FOR CRANIOMAXILLOFACIAL AND ORTHOPEDIC SURGERY**

(30) Priority: 27.05.2025 US 202519218733
(71) Applicant: Techfit Digital Surgery Inc., Daytona Beach, Florida 32114 (US)
(72) Inventor: TORO RESTREPO, Mauricio, Daytona Beach, FL 32114 (US); CORREA PANESSO, Jorge Eduardo, Urbana, IL 61801 (US); GAVIRIA CADAVID, Juan David, Medellín, Antioquia (CO); GUTIÉRREZ URREGO, Juan Camilo, Envigado, Antioquia (CO)
(74) Representative: Boult Wade Tennant LLP

(57) **Abstract**

A computer-implemented method and system for managing a digital academic platform in the field of craniomaxillofacial and orthopedic surgery is provided, wherein the system includes a user management module, a clinical case publishing and dissemination module, an academic interaction engine, and a notification module.

## Description

### Field of technology

The following relates to the field of surgery and surgical planning in maxillofacial and orthopedic procedures. More specifically, the following concerns a computer-implemented method and system for managing a digital academic platform in the field of craniomaxillofacial and orthopedic surgery. In embodiments, the system comprises a user management module (1), a clinical case publication and dissemination module (2), an academic interaction engine (3), and a notifications module (4).

### Background

Historically, medicine and surgery were considered independent disciplines, in which surgeons refined their skills progressively through hands-on experience. This practical knowledge was enriched through accumulated experience and the exchange of expertise among peers. Today, such interactions primarily occur at congresses or specialized meetings, which in many cases are not accessible to everyone or prove insufficient.

In the surgical field, continuous technological advancements have enabled the development of a wide range of tools focused on surgical planning. The use of computer-based tools and other technologies allows for the ongoing optimization and improvement of surgical procedures.

On another front, several tools have been developed to facilitate interaction, management, and collaborative learning among professionals. However, none of these tools are specifically tailored to the fields of craniomaxillofacial or orthopedic surgery.

Within the conventional art, inventions such as the one disclosed in US20240266074A1 exist, which integrates applications, tools, and emerging machine learning techniques in medicine with videoconferencing network technologies in novel business methods that support rapid adaptive learning for medical minds and machines. These methods can leverage domain knowledge and clinical expertise with cognitive networked collaboration, augmented clinical intelligence, and cyber workflows for learning-based healthcare systems. Embodiments of the invention enable multimodal clinical communication, collaboration, consultation, and instruction among heterogeneous teams of people, machines, devices, neural networks, robots, and algorithms, including augmented generative Al algorithms, models, and systems. It supports cognitive enrichment, annotation and labelling, as well as encapsulation, storage, and sharing of image data streams as packaged clinical intelligence.

However, this document presents the drawback of not explicitly addressing applications for surgeons in the craniomaxillofacial or orthopedic specialties, nor does it detail the manner in which interactions are conducted. Additionally, embodiments of the invention focuses on enabling interaction among heterogeneous teams of people, machines, devices, neural networks, robots, and algorithms, including augmented generative AI models and systems.

On the other hand, document US20090307762A1 discloses a system and method for creating and displaying web annotations that are selectively shared within specified online communities. One embodiment of the system and method includes the use of a web browser plug-in that enables a computer user to create content and associate that content with an Internet address in the form of a web note, which is displayed to the user as a presentation layer over the Internet website. This web note is transmitted to a web server that stores the content and attributes of the web note in a database. When that user or another user of the browser plug-in later navigates to the specified Internet address, the web note is retrieved from the database and displayed to the user through the browser in a presentation layer over the Internet website, independently of the website itself, based on user-defined shared content filters.

However, this document presents the disadvantage of not explicitly mentioning 3D models, and it applies broadly to any website.

In turn, document US8924881B2 discloses a method for providing a first dashboard that includes multiple first windows to deliver information about a first set of aspects of a patient's condition. The method also provides a second dashboard that includes multiple second windows to deliver information about a second set of aspects of the patient's condition. Additionally, the method defines a link that enables the second dashboard to be opened upon selecting an element in at least one of the first windows.

However, this document presents the drawback of not referring specifically to the publication or management of surgical cases, nor to a collaborative environment. It does not focus on the step-by-step presentation of surgical procedures, nor does it mention features that enable interaction among surgeons.

Document CN115273583A refers to a multi-person interactive clinical orthopedic teaching method based on mixed reality. The teaching method comprises a plurality of clinical orthopedic teaching modes. A corresponding virtual teaching scene can be automatically generated by selecting the clinical orthopedic teaching mode, and the virtual teaching scene and the user's image information are generated and combined to form an image displayed on a three-dimensional display device. The user uses a hand motion trajectory generation instruction to control the operation of a virtual object in the virtual teaching scene through the interactive device worn by the user, thereby achieving the effect of simulating teaching. The teaching method adopts mixed reality technology to enable interactive teaching among multiple users, including instructors, students, and a mixed reality environment. Through this method, instructors can teach in a virtual orthopedic clinical scene, and students can observe the instructional content and simulated clinical operations in a more intuitive and stereoscopic manner, allowing for greater immersion. At the same time, the teaching locations of instructors are not limited, which greatly enhances the convenience of online education.

However, while the concept of simulating clinical teaching content and displaying clinical operations in a virtual environment is disclosed, embodiments of the invention do not disclose the detailed presentation of clinical cases. Therefore, it is more oriented toward learning and teaching in a clinical training context rather than serving as a platform for sharing and discussing actual executed cases.

Lastly, document US20220039905A1 provides a medical control apparatus comprising: a processing unit configured to cause one or more of a first display region, in which an object related to an input source image is to be displayed; a second display region, in which an object related to an output destination image is to be displayed; and a third display region, in which an object for controlling the input source or output destination is to be displayed, to appear on a screen.

However, this document presents the disadvantage of not specifically referring to the publication or management of surgical cases, nor to a collaborative environment. It does not focus on the step-by-step presentation of surgical procedures, nor does it mention features that would allow surgeons to interact with one another.

### Summary

An aspect relates to a specialized system that enables surgeons to share clinical cases, interact with other professionals, and visualize published cases in 3D with a detailed presentation of the case development, thereby facilitating deep learning and collaboration among surgeons. Currently, surgeons and specialists in these fields do not have access to a platform that efficiently integrates the following:
- A standardized format for documenting clinical cases, including diagnostic images, surgical planning, techniques employed, and postoperative outcomes.
- An interactive 3D visualization system that facilitates anatomical and surgical understanding of the cases through three-dimensional models of anatomical structures and patient-specific medical devices.
- Real-time academic interaction tools that allow feedback among specialists via comments, ratings, and direct contact, promoting scientific collaboration.
- An advanced system for organizing and searching clinical cases, enabling filtering of information by specialty, surgical techniques, and devices used.
- A globally accessible environment where surgeons from different regions can share knowledge without geographical restrictions.

### Brief description

Some of the embodiments will be described in detail, with references to the following Figures, wherein like designations denote like members, wherein:

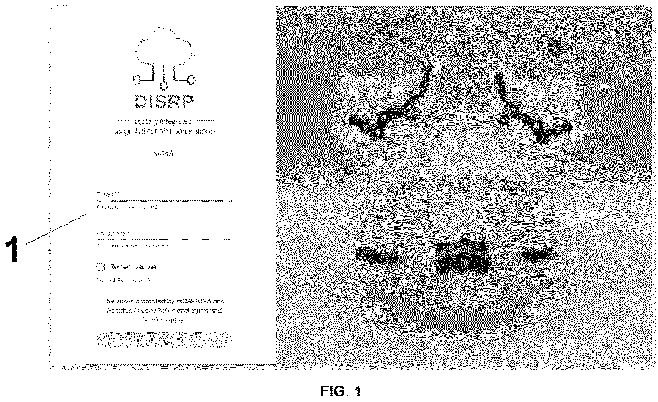

Figure 1 shows a view of the user management module according to embodiments of the present invention;

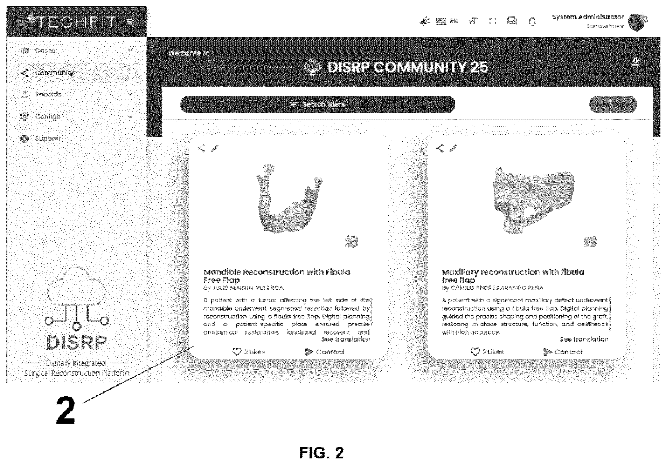

Figure 2 provides a view of the clinical case publication and dissemination module according to embodiments of the present invention;

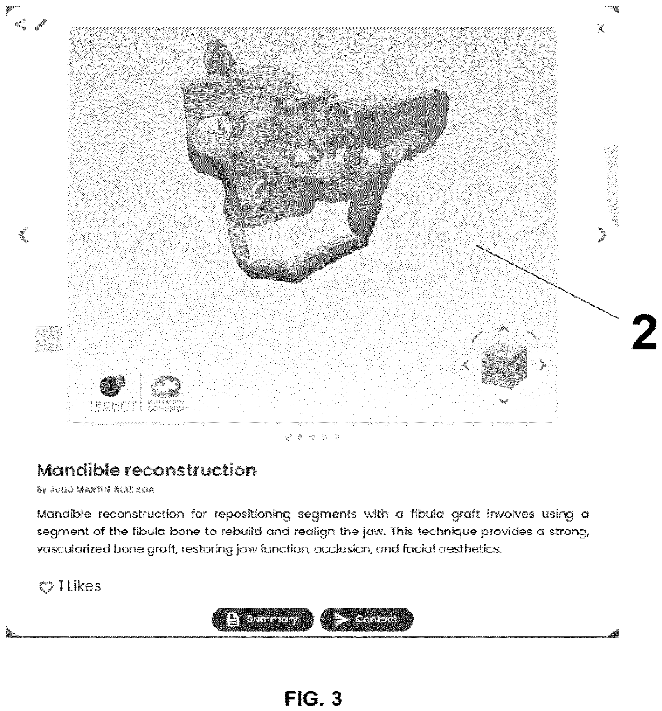

Figure 3 shows a view of the clinical case publication and dissemination module within the interactive visualization environment of three-dimensional (3D) models of anatomical structures and patient-specific medical devices, in accordance with embodiments of the present invention;

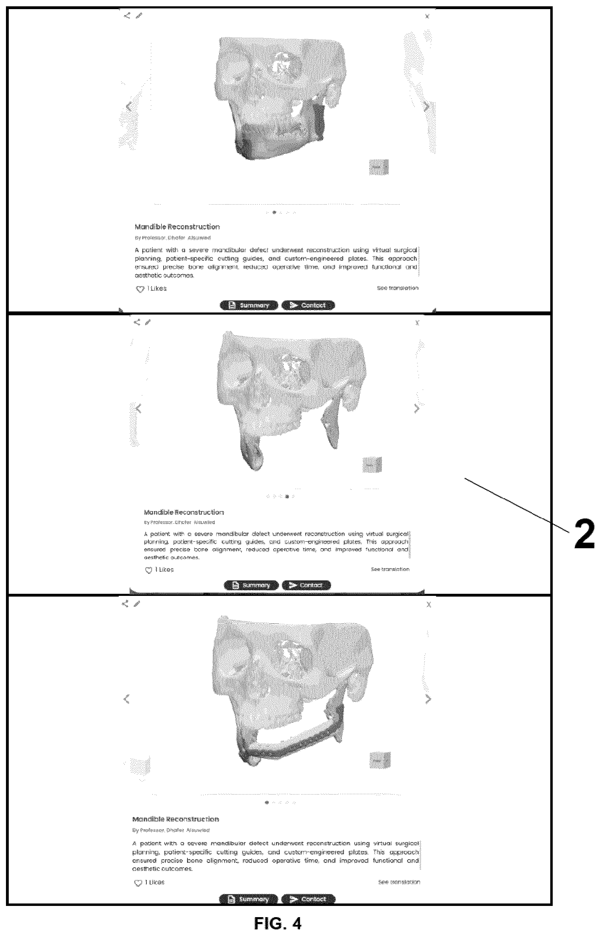

Figure 4 shows a view of the user interface displaying a list of structured clinical cases, including diagnosis, surgical planning, techniques employed, and results obtained, according to embodiments of the present invention;

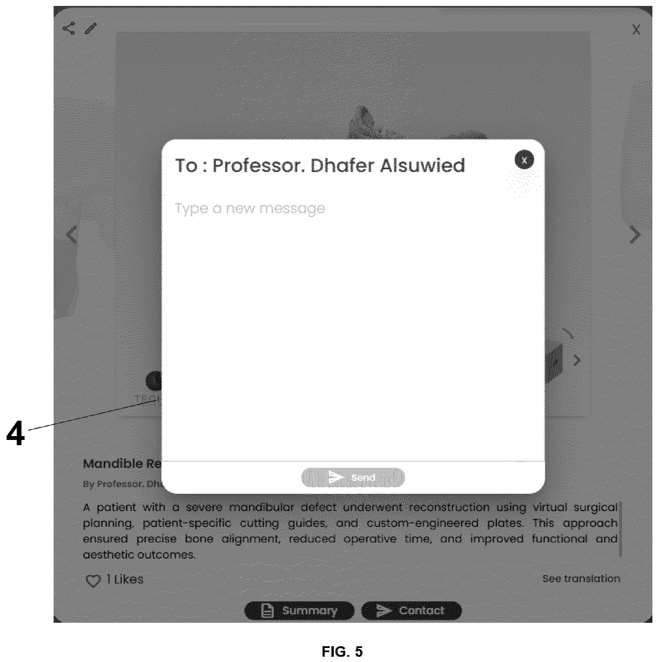

Figure 5 shows a view of the academic interaction engine according to embodiments of the present invention; and

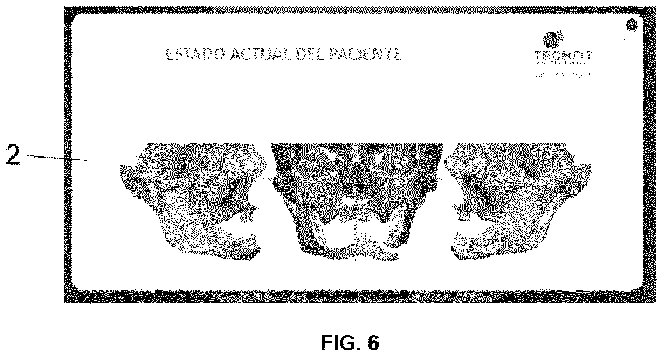

Figure 6 shows a view of the clinical case publication and dissemination module within the interactive visualization environment of three-dimensional (3D) models of anatomical structures and patient-specific medical devices, in accordance with embodiments of the present invention.

### Detailed description

Embodiments of the present invention relate to a computer-implemented method for managing a digital academic platform in the field of craniomaxillofacial and orthopedic surgery, characterized by comprising the following steps:
a. Receiving user credentials for authentication and access to the platform, such as email and password;
b. Providing a user interface that displays a list of structured clinical cases, including diagnosis, surgical planning, techniques used, and obtained outcomes;
c. Enabling interactive visualization of three-dimensional (3D) models of anatomical structures and patient-specific medical devices;
d. Managing interactions between users through comments, academic content rating, and direct contact tools;
e. Facilitating the organization and search of clinical cases through specialized filters and keywords;
f. Recording and storing interaction data to optimize the academic experience and enhance access to information.

Wherein, the interactive visualization of three-dimensional (3D) models comprises:
- The representation of anatomical structures and medical devices in three-dimensional models, allowing rotation, zoom, and change of viewing angles;
- The association of each 3D model with textual information from the corresponding clinical case;
- Enabling comparison between different stages of the surgical procedure.

Wherein the organization and search of clinical cases comprises:
- Uploading of clinical case information by surgeons/users or by the surgical planning team;
- Classification of clinical cases into specific categories according to the surgical specialty;
- Enabling case searches through advanced filters based on keywords, surgical techniques employed, and types of medical devices used.

Wherein the user credentials for authentication and access to the platform comprise an email address and a password, and wherein user access to their profile is protected through a second authentication factor based on temporary access codes sent via SMS or generated by an authenticator application.

Wherein, step a. also comprises: Storing profile information in a relational database accessible through unique identifiers.

Wherein, step d. also comprises: Generating relationships between users through interactions with buttons such as "like," and providing real-time messaging and communication tools between users.

Embodiments of the present invention further comprises a computer-implemented system for real-time academic interaction within a digital platform specialized in craniomaxillofacial and orthopedic surgery, comprising:
- A user management module (1) that enables secure authentication through credentials;
- A clinical case publishing and disclosure module (2) with predefined content structures, where clinical cases are presented in a structured and sequential manner;
- An academic interaction engine (3) that allows users to comment, rate publications, and contact other surgeons;
- A notification module (4) that alerts users about new comments, interactions, and updates on clinical cases of their interest.

Wherein, the structured and sequential presentation of clinical cases comprises:
- The clinical case in a step-by-step format, from obtaining diagnostic images to applying patient-specific medical devices;
- The association of each stage of the procedure with interactive three-dimensional models and additional educational resources, such as diagnostic images, surgical planning, techniques employed, post-operative information, doctor's comments, and others.

Wherein, the system comprises a global access environment from any computing device with internet access, including personal computers and mobile devices, where surgeons from different regions share knowledge without geographical restrictions.

Wherein, the system comprises real-time academic interaction within a digital platform specialized in craniomaxillofacial and orthopedic surgery.

Wherein, the system further comprises a database that stores all the content.

Embodiments of the present invention can be more clearly understood from the following definitions. Concepts not explicitly defined should be interpreted in the broadest sense of their meaning:
Step-by-step presentation of surgical procedures: This concept refers to the stages of surgery related to the medical devices implemented during the surgical intervention, as well as the positioning of such devices, and the required bone cuts and movements to achieve the desired final outcome of the procedure.

Surgical planning: This concept refers to the specific planning related to the medical devices implemented during the surgical intervention, including the positioning of such devices and the bone cuts and movements necessary to obtain the desired final result of the procedure.

Thus, the main advantages of embodiments of the present invention consist of the incorporation of:
- A specialized digital academic platform for craniomaxillofacial and orthopedic surgery, designed for the structured publication of clinical cases.
- An interactive 3D visualization system that allows the observation of three-dimensional models of anatomical structures and patient-specific medical devices.
- A user interface with restricted access through credentials (username and password).
- The structured presentation of clinical cases, including diagnostic images, surgical planning, procedural description, and postoperative outcomes.
- A real-time academic interaction system that allows users to comment, "like," and contact other surgeons for scientific collaboration.
- The organization of clinical cases using advanced filters, enabling searches by surgical specialty and keywords.
- The recording and storage of user interactions to optimize the academic experience and improve accessibility to relevant information.
- A sequential case presentation system that displays the step-by-step development of the surgical procedure.
- Global and real-time access for interaction among surgeons from different regions of the world.

Thus, embodiments of the invention address the fragmentation of information and lack of interactivity in the academic learning and collaboration related to specialized surgical clinical cases, providing a comprehensive digital platform that optimizes documentation, visualization, and discussion of complex medical procedures.

Although the present invention has been disclosed in the form of embodiments and variations thereon, it will be understood that numerous additional modifications and variations could be made thereto without departing from the scope of the invention.

For the sake of clarity, it is to be understood that the use of "a" or "an" throughout this application does not exclude a plurality, and "comprising" does not exclude other steps or elements.

## Claims

1. A computer-implemented method for managing a digital academic platform in the field of craniomaxillofacial and orthopedic surgery, **characterized by** comprising the following steps:
a. receiving user credentials for authentication and access to the platform;
b. providing a user interface that displays a list of structured clinical cases, including diagnosis, surgical planning, techniques used, and outcomes obtained;
c. enabling the interactive visualization of three-dimensional (3D) models of anatomical structures and patient-specific medical devices;
d. managing user interactions through comments, academic content ratings, and direct contact tools;
e. facilitating the organization and search of clinical cases through specialized filters and keywords;
f. recording and storing interaction data to optimize the academic experience and improve access to information.

2. The method according to claim 1, wherein the interactive visualization of three-dimensional (3D) models comprises:
the representation of anatomical structures and medical devices in three-dimensional models, allowing for rotation, zooming, and changes in viewing angles;
the association of each 3D model with textual information from the corresponding clinical case;
allowing comparison between different stages of the surgical procedure.

3. The method according to claim 1, wherein the organization and search of clinical cases comprises:
the upload of information related to clinical cases by surgeons;
the classification of clinical cases into specific categories according to the surgical specialty;
allowing the search for cases through advanced filters based on keywords, surgical techniques used, and type of medical device employed.

4. The method according to claim 1, wherein comprising a digital academic platform in the field of craniomaxillofacial and orthopedic surgery.

5. The method according to claim 1, wherein the user credentials for authentication and access to the platform comprise an email and a password.

6. The method according to claim 1, wherein step a. further comprises:
storing user profile information in a relational database accessible through unique identifiers.

7. The method according to claim 1, wherein step d. further comprises:
generating relationships between users through the interaction with buttons such as "like," and providing messaging and real-time communication tools between users.

8. A computer-implemented system for academic interaction within a digital platform specialized in surgery, comprising:
a user management module that enables secure authentication through credentials;
a clinical case publishing and dissemination module with predefined content structures, wherein clinical cases are presented in a structured and sequential manner;
an academic interaction engine that allows users to comment on, rate publications, and contact other surgeons;
a notification module that alerts users to new comments, interactions, and updates in clinical cases of interest.

9. The system according to claim 8, wherein the structured and sequential presentation of clinical cases comprises:
the clinical case in a step-by-step format, from obtaining diagnostic images to the application of personalized medical devices;
the association of each stage of the procedure with interactive three-dimensional models and additional educational resources, such as diagnostic images, surgical planning, techniques used, postoperative information, physician comments, among others.

10. The system according to claim 8, wherein it comprises a global environment accessible from any computing device with internet access, where surgeons from different regions share knowledge without geographic restrictions.

11. The system according to claim 8, wherein it comprises real-time academic interaction within a digital platform specialized in craniomaxillofacial and orthopedic surgery.
